# EUROPEAN PATENT SPECIFICATION

(11) **EP 1 883 365 B1**
(45) Date of publication and mention of the grant of the patent: **14.11.2018**
(21) Application number: 06760025.4
(22) Date of filing: 16.05.2006
(51) Int. Cl.: A61B 18/14

(54) **CONDUCTIVE FLUID BRIDGE ELECTROSURGICAL APPARATUS**
GERÄT FÜR DIE ELEKTROCHIRURGIE MIT EINER BRÜCKE MIT LEITFÄHIGER FLÜSSIGKEIT
APPAREIL ELECTROCHIRURGICAL A PONT DE FLUIDE CONDUCTEUR

(30) Priority: 16.05.2005 US 130931
(43) Date of publication of application: 06.02.2008
(73) Proprietor: ArthroCare Corporation, Austin, TX 78735 (US)
(72) Inventor: DAHLA, Robert H., Sunnyvale, CA 94087 (US)
(74) Representative: Mathys & Squire LLP
(86) International application number: PCT/US2006/019095
(87) International publication number: WO 2006/125007

(56) References cited:
- WO-A2-03/024305
- US-A1- 2002 095 152
- US-B2- 6 837 887

## Description

### BACKGROUND

### Field of the Invention

This invention relates to an electrosurgical apparatus comprising an active electrode, a return electrode, and a conductive fluid bridge maintained between the electrodes during use regardless of the orientation of the apparatus.

### Description of the Prior Art

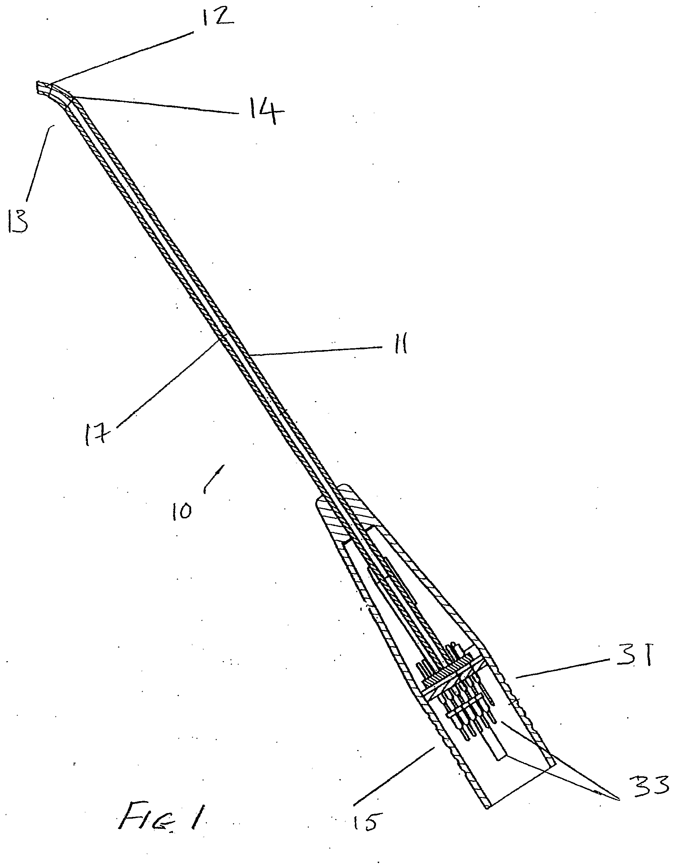

In some electrosurgical procedures an instrument, as illustrated for example in Fig. 1, comprising an active electrode (12) and a return electrode (14) is used to treat body tissue. Treatment includes without limitation coagulation, cutting, ablating, abrading or puncturing the tissue. In various embodiments, generating plasma between the electrodes and applying the plasma to the tissue, without passing a current through the tissue, effects treatment. Examples of these instruments and their use in electrosurgical procedures are described in U.S. Patent Nos. 5,683,366 and 6,235,020 herein incorporated by reference. In various embodiments, the conductive fluid path is an electrolyte e.g., saline, lactated ringers solution, or conductive gels, and one electrode, referred to as the active electrode, is designed to generate a higher current density relative to other electrode, referred to as the return electrode. The source of the current is a high frequency voltage applied across the electrodes.

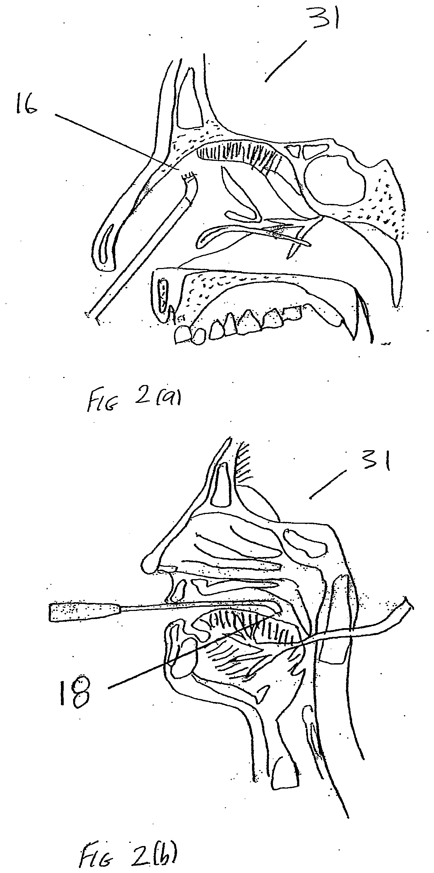

With these instruments, for certain procedures it is a problem to maintain an unbroken conductive fluid path between the electrodes during use. For example as shown in Figure 2(a), when the instrument is being used to treat tissues (16) in the nose, gravity tends to cause the fluid bridge to break. Similarly, as shown in Figure 2(b) the fluid bridge is easily broken when the electrodes (18) are oriented downwards during use. In these procedures because the distal end of the instrument is positioned either higher than the proximal end and/or the electrodes are turned up during use, gravity causes the fluid to flow downwards and away from the electrodes, thus breaking the fluid path. With the breaking of the fluid path, the instrument may exhibit undesirable thermal generation as opposed to the desired generation of plasma.

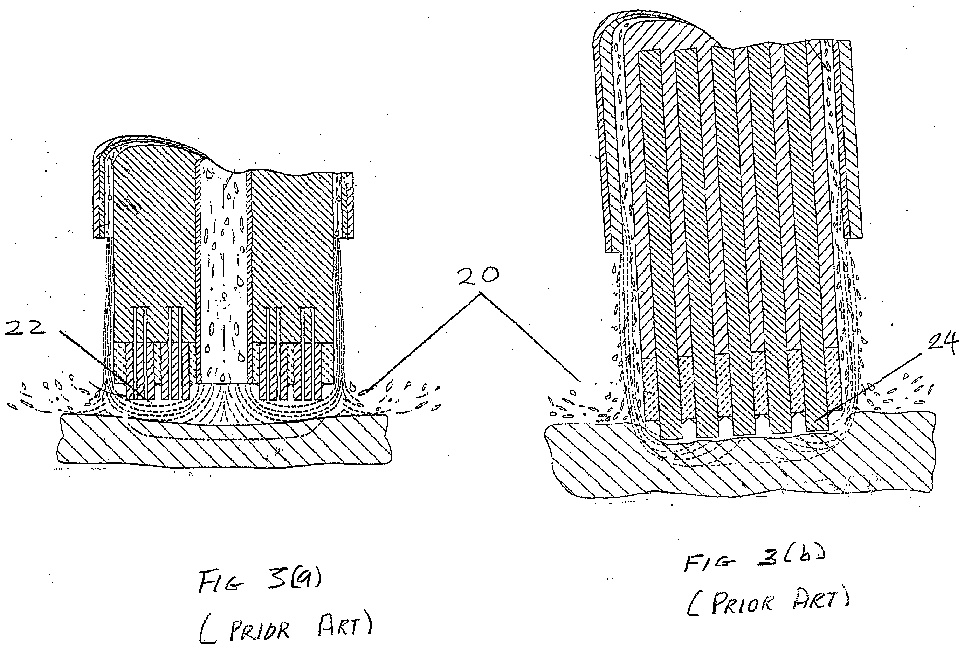

An approach to maintaining the conductive fluid path during use regardless of the orientation of the instrument is to direct a flow of conductive fluid between the electrodes such that the electrodes and or the tissues are always flooded. This is illustrated for example in Figs. 3 (a) and (b) wherein a stream of fluid (20) bathes or floods the electrodes (22, 24) during use. In various embodiments, the fluid is discharged from an annular member onto the tissue and/or between the electrodes. In various embodiments, the annular member is an open-ended tube (17) disposed within the instrument as is shown for example in Fig 1; in other embodiments, the annular member resides externally on an elongate member (11) of the instrument. In various embodiments a vacuum is positioned near the fluid path to aspirate excess fluid during use.

United States Patent Application Publication US2002/0095152 provides an electrosurgical probe which includes a shaft and a handle together with a fluid delivery element and an aspiration unit in which an active electrode includes a cross piece located distal to an aspiration port and a fluid delivery element or unit including an outer sheath.

While the fluid path from an annular member is relative easy to maintain by flooding the tissue and/or by ensuring that the tissue is below the level of the electrodes, in using the instruments where flooding is not possible and/or desired as, for example in treating the larynx and the nose, this approach is unsatisfactory. Further, the flooding may be undesirable if it obstructs the line of sight to the tissue during use.

Accordingly, in view of these deficiencies and also in view of the desire to improve the instrument, it is an objective of this invention to provide an instrument wherein the conductive fluid path is maintained during use regardless of the orientation of the electrodes relative to the tissue.

WO03/024305 describes a method for treating a target tissue in which an active electrode terminal of an electrosurgical probe is positioned in at least close proximity to the target tissue. A high frequency voltage is then applied between the active electrode and a return electrode, wherein, the high frequency voltage is sufficient to volumetrically remove (ablate), sever, or modify at least a portion of the target tissue. The probe comprises an electrode assembly including an electrode support and at least one active electrode terminal arranged within the electrode support, the electrode support having a suction cavity, and each active electrode terminal having an electrode lumen therein, the electrode lumen in communication with the suction cavity. Unwanted materials may be aspirated from a surgical site via the electrode lumen.

### SUMMARY OF THE INVENTION

In one embodiment, the apparatus comprises an electrosurgical instrument for treating body tissue, comprising an active and a return electrode, a vacuum suction inlet located near the active electrode, and
a pinhole near the return electrode such that the pinhole and the vacuum suction cooperate to maintain a conductive fluid bridge between the active and return electrodes during use, regardless of the orientation of the instrument relative to the tissue.

In another embodiment the apparatus is an electrosurgical instrument comprising an elongated shaft having a distal end portion, an active and a return electrode disposed on the distal end portion, a vacuum system having a suction inlet near the active electrode, and at least one pinhole near the return electrode for forming an conductive fluid bridge between the active and return electrodes during use regardless of the orientation of the instrument.

In another embodiment the invention is a method of ablating body tissue, comprising applying ablative energy to a target tissue not flooded or submerged with an electrically conductive fluid; maintaining an electrically conductive fluid bridge between an active and a return electrode near the target tissue to generate the ablative energy regardless of the orientation of the electrodes relative to the target tissue. In one embodiment the conductive fluid bridge is maintained during use by causing the fluid to flow out of a pinhole placed on the distal end of a shaft towards an aspiration port on the shaft.

In another embodiment the invention is a system for ablating tissue comprising an apparatus including a pinhole for maintaining an electrically conductive fluid bridge between an active and a return electrode on the apparatus regardless of the orientation of the electrodes relative to the tissue; a vacuum system for aspirating fluid from the fluid bridge; a high-frequency voltage generator for generating plasma between the active and return electrodes; and a conductive fluid reservoir system for maintaining a supply of conductive fluid at the electrodes.

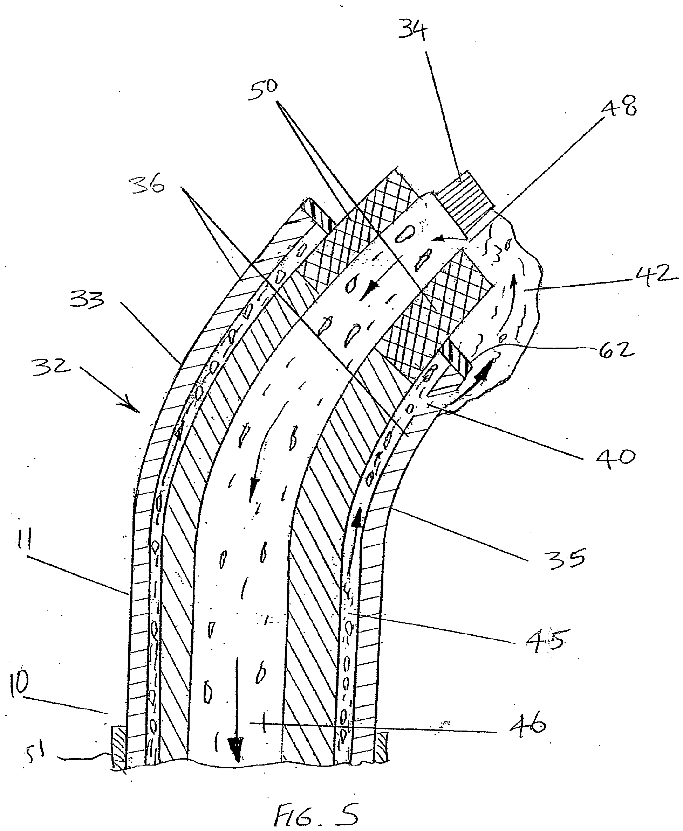

In the various embodiments, the instrument maintains a conductive fluid bridge between the electrodes during use regardless of the orientation of the electrodes relative to the target tissue. The fluid bridge comprises an electrically conductive fluid in the form of a droplet, a fluid channel, a glob of gel between the electrodes, etc.. An example of a fluid bridge in accordance with the present invention is illustrated in Fig. 5 wherein a bridge (42) is illustrated between the active electrode (34) and a return electrode (36). By directing the conductive fluid through a pinhole near the electrodes, and by operating vacuum aspiration port near the electrodes in accordance with the invention, a conductive fluid bridge of a proper size is maintained during use regardless of the orientation of the instrument relative to the tissue.

Advantageously, since the pinhole of the present instrument restricts the amount of fluid forming the fluid bridge during use, the size of the fluid bridge is restricted. Thus with the vacuum suction inlet located on the instrument, in use the fluid bridge is maintained regardless of the orientation of the instrument with respect to the tissue, without flooding the electrodes and/or the tissue. Accordingly the present instrument is usable to treat tissue for any orientation of the electrodes relative to the tissue, without loss of plasma generation. Additionally the instrument can be used in procedures wherein it is neither desirable nor possible to flood the tissue. Further, since the pinhole restricts the amount of conductive fluid between the electrodes compared to the amount of fluid from an annular orifice on conventional instruments, the instrument allows for better visibility during use.

Without desiring to be bound to any explanation for the results achieved, it is believed that in accordance with the present instrument since fluid emerging from the pinhole creates a small fluid bridge between the electrodes, the surface tension forces arising from the geometry of the instrument and the fluid, in combination with negative pressure of the vacuum and the fluid momentum, counteract the effect of gravity such that the fluid bridge is maintained between the electrodes during use, regardless of the orientation of the instrument.

### BRIEF DESCRIPTION OF THE DRAWINGS

Fig. 1 is a perspective view of an embodiment of an electrosurgical apparatus.
Fig. 2a and 2b are cross-sectional views of target tissue treatable with an electrosurgical apparatus.
Fig. 3a and 3b are cross-section views of prior art instruments illustrating a supply of conductive fluid to the electrodes.
Fig. 4 is a perspective view of an environment for using the present apparatus.
Fig 5 is a cross-sectional view of an embodiment of a pinhole for forming a fluid bridge between the electrodes without flooding.

### DETAILED DESCRIPTION OF A PREFERRED EMBODIMENT

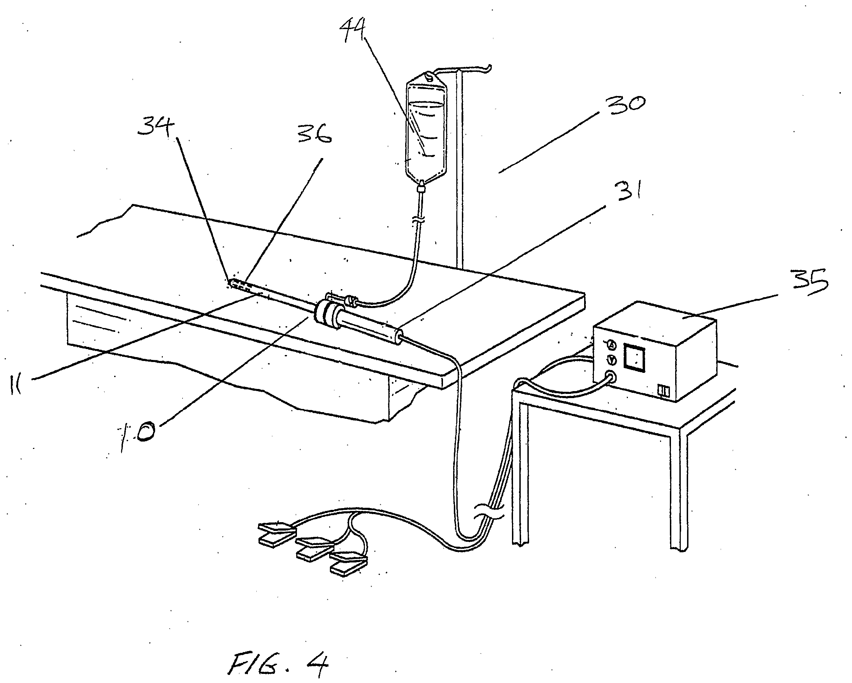

Embodiments of the present invention are illustrated in Figs. 1, 2a, 2b, 4 and 5 wherein a pinhole is provided for controlling the supply of fluid to form a fluid bridge between the electrodes. In the embodiments illustrated in Figs.1 and 4, the instrument includes an elongated member (11) having a proximal end portion (15, 31) that includes electrical terminals (33) for connecting the active and return electrodes (12, 14, 34, 36) to a high frequency voltage supply (35).

In the embodiment illustrated in Fig. 5, the instrument (10) comprises an elongated member (11) having a distal end portion (32); an active electrode (34) and a return electrode (36) disposed on the distal end portion; and at least one pinhole (40) defined on the distal end portion for forming an conductive fluid bridge (42) between the active and return electrodes. Also in the embodiment of Fig. 5, the distal end portion (32) is a generally curved member having an outer curved surface (33) and an inner curved surface (35). In this embodiment, a pinhole (40) is defined by the inner curved surface. Also included in this embodiment is an electrically insulating member (50) disposed between the active and return electrodes. A vacuum system (46) is provided within a lumen in the elongated member (11) for aspirating fluid through a suction inlet (48) away from the active and return electrodes (34, 36). In alternative embodiments not shown, the pinhole is defined on the outer curved surface (33) through the insulating member (50). Also, an outer insulating layer (51), member or cover may be provided on return electrode 36.

Also in the embodiment of Fig. 5, the pinhole (40) is aimed to discharge fluid towards the active electrode (34) near the return electrode (36). In this embodiment, the conductive fluid is saline or lactated ringers solution, and the fluid bridge is in the form of a bridge (42) between the active and return electrodes (32) is formed without flooding the target location, the electrodes or any other area. In this embodiment the conductive fluid bridge (42) forms a conductive path between the active (34) and return (36) electrodes regardless of the orientation of the distal end portion (32) relative to the target location as illustrated in Figs.2 (a) and 2(b). Although saline is shown in this embodiment, other conventional conductive fluids can be used including gels and lactated ringers solution.

The shape of the pinhole may vary widely. For example it may be circular, square, or another shape. Also the number of pinholes may vary from one to about 5-10 or more. Further, the angle or direction of the fluid through the exit of the pinhole is preferably towards the active electrode, which is generally distal relative to the return electrode. In various embodiments, the angle of the hole relative to the shaft is preferably less than 90° and is about 30-60° from the longitudinal axis of the shaft member. In various embodiments, a range of pinhole sizes is useable, however a preferred range is a diameter between about 0.015 inch to about 0.250 inch, and more preferred about 0.030 inch.

In the embodiment illustrated in Figs. 4 and 5., the apparatus further includes a conductive fluid reservoir (44) connected to the pinhole (40) through a lumen (45) in the elongated shaft for supplying conductive fluid to the pinhole. The lumen may be sealed at the distal end with an endcap (62) adhesive, ceramic, epoxy or another suitable means. In the configuration shown in Figs.4 and 5 the conductive fluid reservoir (44) height relative to the position of the distal tip of the apparatus (34) is adjustable to control the formation of the conductive fluid droplet. This height may vary preferably from about 5 to 3000 cm, more preferably from about 40 to about 100 cm.

In the embodiment of Fig. 5, the apparatus further comprises a vacuum system (46) defining a suction inlet (48) on the end portion. The vacuum system (46) in this embodiment is disposed at least partly within a lumen defined by the elongated member (11). In this embodiment a conventional vacuum system as disclosed in U.S. Patent Nos. 5,683,366 and 6,235,020, supra, can be used.

Also as shown in Fig. 4, this embodiment of the apparatus includes a high frequency voltage supply connected to the active (34) and return (36) electrodes. Examples of various configuration of voltage supply are described in U.S. Patent Nos. 5,683,366 and 6,235,020, incorporated herein, supra. An example of a voltage supply is the Coblator 2 controller manufactured by ArthroCare Corporation.

In a further embodiment, the invention is a method of performing a dry field surgical procedure comprising: applying ablative energy from a distal end of an elongated shaft towards a target tissue wherein the target tissue is not flooded or submerged in electrically conductive fluid; and maintaining a fluid bridge between an active electrode and a return electrode at the distal end of the shaft regardless of orientation of the electrodes. The fluid bridge is maintained by directing conductive fluid through a pinhole defined on the distal end of the shaft in accordance with the present apparatus. In various embodiments, the present method further includes generating the plasma from the electrically conductive fluid by applying a conventional high frequency voltage across the electrodes, as described, for example, in U.S. Patent Nos. 5,683,366 and 6,235,020, supra.

In accordance with the present method, since the apparatus maintains an electrically conductive fluid bridge between the electrodes regardless of the orientation of the electrodes, the instrument can be used in dry field procedures for ablation tissues such as in the larynx, the nose, the adenoids and tonsils, as described, supra.

While the invention is described with reference to the Figures and method herein, it will be appreciated by one ordinarily skilled in the art that the invention can also be practiced with obvious modifications wherein it is desired to treat tissue using an conductive fluid bridge. Thus the scope of the invention should not be limited to the embodiments as described herein, but is limited only by the scope of the appended claims.

## Claims

1. An electrosurgical apparatus for treating targeted body tissue, comprising: an active and a return electrode; a vacuum suction inlet for conductive fluid located near said active electrode; and at least one pinhole defined by said return electrode, wherein said pinhole and said vacuum suction inlet are adapted to maintain an electrically conductive fluid bridge between said active and return electrodes regardless of the orientation of said apparatus, wherein the pinhole is aimed to discharge the conductive fluid toward said active electrode to form said conductive fluid bridge with said return electrode.

2. The apparatus of claim 1 comprising:
an elongated shaft having a distal end portion in which the active electrode and the return electrode are disposed on said distal end portion; a vacuum system; wherein said pinhole is defined by said distal end portion near said return electrode

3. The apparatus of claim 2, further comprising a conductive fluid system for supplying said conductive fluid to said pinhole.

4. The apparatus of claim 1 or 2, wherein said pinhole is about 0.015 inch to about 0.205 inch in diameter.

5. The apparatus of claim 1 or 2, wherein said pinhole is about 0.030 inch in diameter.

6. The apparatus of claim 1, further comprising an elongated member having distal end portion comprised of said active and return electrodes and including an outer and an inner curved sections, wherein said pinhole is defined by said inner curved section.

7. The apparatus of claim 1, further comprising an elongated member having a distal end portion comprised of said active and return electrodes and including an outer and an inner curved section, wherein said pinhole is defined by said outer curved section.

8. The apparatus of claim 1 or 2, wherein said conductive fluid bridge is selected from the group consisting of saline and lactated ringers solution.

9. The apparatus of claim 1, further comprising a fluid reservoir fluidly connected to said pinhole.

10. The apparatus of claim 9, wherein said fluid reservoir is disposed at least partly within a lumen defined by said apparatus.

11. The apparatus of claim 1, wherein said vacuum system is disposed at least partly within a lumen defined by said apparatus.

12. The apparatus of claim 6 wherein an insulating member (50) is disposed between the active and return electrodes.

13. The apparatus of claim 12, wherein said pinhole is positioned to discharge said conductive fluid across said insulating member to said active electrode such that said conductive fluid provides a conductive bridge between said electrodes across said insulating member.

14. The apparatus of claim 1 or 2, further including a high frequency voltage supply connected to said active and return electrodes for generating plasma from said conductive fluid bridge.

15. The apparatus of claim 14, wherein said active and return electrodes are adapted to ablate body tissues in the larynx and nose.

16. The apparatus of claim 2, wherein said pinhole is defined in said distal end portion at an angle of less than 90° to the longitudinal axis of said distal end portion.

17. The apparatus of claim 2, wherein said pinhole is defined in said distal end portion at an angle of about 30° to 60° to the longitudinal axis of said distal end portion.

18. The apparatus of claim 2, wherein said vacuum system is disposed at least partly within a lumen defined by said distal end portion.

19. The apparatus of claim 2, wherein said conductive fluid system is disposed at least partly within a lumen defined by said distal end portion.

20. The apparatus of claim 1 or 2, further comprising a high frequency voltage supply for generating plasma between said active and return electrodes.

21. The apparatus of claim 1 or 2, further comprising an electrically insulating member disposed between said active and return electrodes.

22. A system for ablating tissue comprising: an apparatus according to any preceding claim;
a vacuum system for aspirating fluid from said fluid bridge; a high-frequency voltage generator for generating plasma between said active and return electrodes; and a conductive fluid reservoir system for maintaining a supply of said conductive fluid at said electrodes.

## Patentansprüche

1. Elektrochirurgische Vorrichtung zum Behandeln von gezieltem Körpergewebe, die Folgendes umfasst: eine aktive und eine Rückführungselektrode; einen in der Nähe der aktiven Elektrode angeordneten Vakuumsaugeinlass für leitfähige Flüssigkeit; und mindestens ein Nadelloch, das durch die Rückführungselektrode definiert ist, wobei das Nadelloch und der Vakuumsaugeinlass dazu ausgelegt sind, eine elektrisch leitfähige Flüssigkeitsbrücke zwischen der aktiven und der Rückführungselektrode ungeachtet der Orientierung der Vorrichtung aufrechtzuerhalten, wobei das Nadelloch darauf abgezielt ist, die leitfähige Flüssigkeit in Richtung der aktiven Elektrode abzulassen, um die leitfähige Flüssigkeitsbrücke mit der Rückführungselektrode zu bilden.

2. Vorrichtung nach Anspruch 1, die Folgendes umfasst:
einen länglichen Schaft mit einem distalen Endteil, in dem die aktive Elektrode und die Rückführungselektrode auf dem distalen Endteil angeordnet sind; ein Vakuumsystem; wobei das Nadelloch durch den distalen Endteil in der Nähe der Rückführungselektrode definiert wird.

3. Vorrichtung nach Anspruch 2, die ferner ein leitfähiges Flüssigkeitssystem zum Zuführen der leitfähigen Flüssigkeit zu dem Nadelloch umfasst.

4. Vorrichtung nach Anspruch 1 oder 2, wobei das Nadelloch einen Durchmesser von etwa 0,015 Zoll bis etwa 0,205 Zoll hat.

5. Vorrichtung nach Anspruch 1 oder 2, wobei das Nadelloch einen Durchmesser von etwa 0,030 Zoll hat.

6. Vorrichtung nach Anspruch 1, die ferner ein längliches Element mit einem distalen Endteil hat, das aus der aktiven und der Rückführungselektrode besteht und einen äußeren und einen inneren gekrümmten Abschnitt aufweist, wobei das Nadelloch durch den inneren gekrümmten Abschnitt definiert ist.

7. Vorrichtung nach Anspruch 1, die ferner ein längliches Element mit einem distalen Endteil hat, das aus der aktiven und der Rückführungselektrode besteht und einen äußeren und einen inneren gekrümmten Abschnitt aufweist, wobei das Nadelloch durch den inneren gekrümmten Abschnitt definiert ist.

8. Vorrichtung nach Anspruch 1 oder 2, wobei die leitfähige Flüssigkeitsbrücke aus der Gruppe ausgewählt wird, die aus Salzlösung und Ringer-Laktat-Lösung besteht.

9. Vorrichtung nach Anspruch 1, die ferner einen Flüssigkeitsbehälter umfasst, der in Fluidverbindung mit dem Nadelloch steht.

10. Vorrichtung nach Anspruch 9, wobei der Flüssigkeitsbehälter zumindest teilweise innerhalb eines Hohlraums angeordnet ist, der von der Vorrichtung definiert ist.

11. Vorrichtung nach Anspruch 1, wobei das Vakuumsystem zumindest teilweise innerhalb eines Hohlraums angeordnet ist, der von der Vorrichtung definiert ist.

12. Vorrichtung nach Anspruch 6, wobei ein Isolierelement (50) zwischen der aktiven und der Rückführungselektrode angeordnet ist.

13. Vorrichtung nach Anspruch 12, wobei das Nadelloch positioniert ist, um die leitfähige Flüssigkeit über das Isolierelement zu der aktiven Elektrode abzulassen, so dass die leitfähige Flüssigkeit eine leitfähige Brücke zwischen den Elektroden über das Isolierelement bereitstellt.

14. Vorrichtung nach Anspruch 1 oder 2, die ferner eine Hochfrequenz-Spannungsversorgung aufweist, die mit der aktiven und Rückführungselektrode verbunden ist, um Plasma von der leitfähigen Flüssigkeitsbrücke zu erzeugen.

15. Vorrichtung nach Anspruch 14, wobei die aktive und die Rückführungselektrode dazu ausgelegt sind, Körpergewebe in Kehlkopf und Nase abzutragen.

16. Vorrichtung nach Anspruch 2, wobei das Nadelloch in dem distalen Endteil bei einem Winkel von weniger als 90° zur Längsachse des distalen Endteils definiert ist.

17. Vorrichtung nach Anspruch 2, wobei das Nadelloch in dem distalen Endteil bei einem Winkel von etwa 30° bis 60° zur Längsachse des distalen Endteils definiert ist.

18. Vorrichtung nach Anspruch 2, wobei das Vakuumsystem zumindest teilweise innerhalb eines Hohlraums angeordnet ist, der von dem distalen Endteil definiert ist.

19. Vorrichtung nach Anspruch 2, wobei das leitfähige Flüssigkeitssystem zumindest teilweise innerhalb eines Hohlraums angeordnet ist, der von dem distalen Endteil definiert ist.

20. Vorrichtung nach Anspruch 1 oder 2, die ferner eine Hochfrequenz-Spannungsversorgung aufweist, um Plasma zwischen der aktiven und der Rückführungselektrode zu erzeugen.

21. Vorrichtung nach Anspruch 1 oder 2, die ferner ein elektrisches Isolierelement aufweist, das zwischen der aktiven und der Rückführungselektrode angeordnet ist.

22. System zum Abtragen von Gewebe, das Folgendes umfasst: eine Vorrichtung gemäß einem beliebigen vorausgehenden Anspruch;
ein Vakuumsystem zum Absaugen von Flüssigkeit von der Flüssigkeitsbrücke; einen Hochfrequenz-Spannungsgenerator zum Erzeugen von Plasma zwischen der aktiven und der Rückführungselektrode; und ein leitfähiges Flüssigkeitsbehältersystem zum Aufrechterhalten einer Zufuhr der leitfähigen Flüssigkeit an den Elektroden.

## Revendications

1. Appareil électrochirurgical destiné à traiter un tissu corporel ciblé, comprenant : une électrode active et une électrode de retour ; une entrée d'aspiration pour fluide conducteur située près de ladite électrode active ; et au moins un trou d'épingle défini par ladite électrode de retour, ledit trou d'épingle et ladite entrée d'aspiration étant adaptés pour maintenir un pont de fluide électriquement conducteur entre lesdites électrodes active et de retour indépendamment de l'orientation dudit appareil, le taux d'épingle étant destiné à déverser le fluide conducteur vers ladite électrode active pour former ledit pont de fluide conducteur avec ladite électrode de retour.

2. Appareil de la revendication 1 comprenant :
une tige allongée ayant une partie d'extrémité distale, l'électrode active et l'électrode de retour étant disposées sur ladite partie d'extrémité distale ; un système de vide ; ledit trou d'épingle étant défini par ladite partie d'extrémité distale près de ladite électrode de retour.

3. Appareil de la revendication 2, comprenant en outre un système de fluide conducteur destiné à fournir ledit fluide conducteur audit trou d'épingle.

4. Appareil de la revendication 1 ou 2, dans lequel ledit trou d'épingle fait environ 0,015 pouce à environ 0,205 pouce de diamètre.

5. Appareil de la revendication 1 ou 2, dans lequel ledit trou d'épingle fait environ 0,030 pouce de diamètre.

6. Appareil de la revendication 1, comprenant en outre un élément allongé ayant une partie d'extrémité distale composée desdites électrodes active et de retour et comportant des sections incurvées externe et interne, ledit trou d'épingle étant défini par ladite section incurvée interne.

7. Appareil de la revendication 1, comprenant en outre un élément allongé ayant une partie d'extrémité distale composée desdites électrodes active et de retour et comportant une section incurvée externe et interne, ledit trou d'épingle étant défini par ladite section incurvée externe.

8. Appareil de la revendication 1 ou 2, dans lequel ledit pont de fluide conducteur est choisi dans le groupe constitué par une solution saline et une solution lactée de Ringer.

9. Appareil de la revendication 1, comprenant en outre un réservoir de fluide fluidiquement relié audit trou d'épingle.

10. Appareil de la revendication 9, dans lequel ledit réservoir de fluide est disposé au moins partiellement à l'intérieur d'une lumière définie par ledit appareil.

11. Appareil de la revendication 1, dans lequel ledit système de vide est disposé au moins partiellement à l'intérieur d'une lumière définie par ledit appareil.

12. Appareil de la revendication 6 dans lequel un élément isolant (50) est disposé entre les électrodes active et de retour.

13. Appareil de la revendication 12, dans lequel ledit trou d'épingle est positionné pour déverser ledit fluide conducteur à travers ledit élément isolant jusqu'à ladite électrode active de telle sorte que ledit fluide conducteur fournit un pont conducteur entre lesdites électrodes à travers ledit élément isolant.

14. Appareil de la revendication 1 ou 2, comportant en outre une source de tension à haute fréquence reliée auxdites électrodes active et de retour pour générer un plasma à partir dudit pont de fluide conducteur.

15. Appareil de la revendication 14, dans lequel lesdites électrodes active et de retour sont adaptées pour l'ablation de tissu corporel dans le larynx et le nez.

16. Appareil de la revendication 2, dans lequel ledit trou d'épingle est défini dans ladite partie d'extrémité distale à un angle de moins de 90° par rapport à l'axe longitudinal de ladite partie d'extrémité distale.

17. Appareil de la revendication 2, dans lequel ledit trou d'épingle est défini dans ladite partie d'extrémité distale à un angle d'environ 30° à 60° par rapport à l'axe longitudinal de ladite partie d'extrémité distale.

18. Appareil de la revendication 2, dans lequel ledit système de vide est disposé au moins partiellement à l'intérieur d'une lumière définie par ladite partie d'extrémité distale.

19. Appareil de la revendication 2, dans lequel ledit système de fluide conducteur est disposé au moins partiellement à l'intérieur d'une lumière définie par ladite partie d'extrémité distale.

20. Appareil de la revendication 1 ou 2, comprenant en outre une source de tension à haute fréquence pour générer un plasma entre lesdites électrodes active et de retour.

21. Appareil de la revendication 1 ou 2, comprenant en outre un élément électriquement isolant disposé entre lesdites électrodes active et de retour.

22. Système d'ablation de tissu comprenant : un appareil selon une quelconque revendication précédente ;
un système de vide pour aspirer un fluide depuis ledit pont de fluide ; un générateur de tension à haute fréquence pour générer un plasma entre lesdites électrodes active et de retour ; et un système de réservoir de fluide conducteur pour maintenir une fourniture dudit fluide conducteur au niveau desdites électrodes.
